# EUROPÄISCHE PATENTSCHRIFT

(11) **EP 3 285 070 B1**
(45) Veröffentlichungstag und Bekanntmachung des Hinweises auf die Patenterteilung: **26.02.2020**
(21) Anmeldenummer: 17180435.4
(22) Anmeldetag: 10.07.2017
(51) Int. Cl.: G01N 33/487, G01N 33/18, A61L 2/08, A61L 2/28, C12M 1/36, A61L 2/00

(54) **AUTONOME VORRICHTUNG ZUM ERFASSEN VON EIGENSCHAFTEN EINES MESSMEDIUMS UND VERFAHREN DAFÜR**
AUTONOMOUS DEVICE FOR DETECTING THE CHARACTERISTICS OF A MEASUREMENT MEDIUM AND METHOD FOR SAME
DISPOSITIF AUTONOME DESTINÉ À DÉTECTER DES PROPRIÉTÉS D'UN MILIEU DE MESURE ET PROCÉDÉ ASSOCIÉ

(30) Priorität: 19.08.2016 DE 102016115403
(43) Veröffentlichungstag der Anmeldung: 21.02.2018
(73) Patentinhaber: Hamilton Bonaduz AG, 7402 Bonaduz (CH)
(72) Erfinder: Juillerat, Frédéric, 7417 Paspels (CH); Gasteuil, Yoann, 69007 Lyon (FR)
(74) Vertreter: Caspary, Karsten

(56) Entgegenhaltungen:
- DE-A1-102008 052 693
- US-A1- 2009 273 447
- US-A1- 2014 062 669
- US-A1- 2015 323 486

## Beschreibung

Die vorliegende Erfindung betrifft eine autonome Vorrichtung zum Erfassen von Eigenschaften eines Messmediums sowie ein Verfahren dafür. Insbesondere betrifft die vorliegende Erfindung eine Sensoreinheit zur Messung von Eigenschaften eines Messmediums, die bei vielen Prozessen in der biopharmazeutischen, pharmazeutischen, biotechnologischen und chemischen Industrie Verwendung findet.

Bei solchen Prozessen werden Sensoren verwendet, mit denen eine Vielzahl von Prozessparametern und -eigenschaften zu einem beliebigen Prozesszeitpunkt gemessen werden kann. Ähnlich wie die in zunehmendem Maße verwendeten Einmalartikel bzw. Einwegartikel wie beispielsweise Behälter und Pipetten aus Kunststoff, die eingesetzt werden, um bei derartigen Prozessen die Produktionsmenge und -sicherheit zu erhöhen sowie die Herstellungskosten und -zeiten so gering wie möglich zu halten, werden diese Sensoren häufig nur einmal benutzt. Dabei müssen sie wie Einmalartikel bei ihrer Verwendung frei von Mikroorganismen bzw. steril sein. Ein Standardverfahren zur Sterilisierung ist die sogenannte Gamma-Sterilisierung, d. h. die Sterilisierung mittels radioaktiver Gamma-Strahlung.

Gamma-Strahlung ist die Anwendung elektromagnetische Strahlen (Gammastrahlen), die von Radionukleiden wie z.B. Cobalt 60 Isotopen (60 Co bzw. ⁶⁰Co) und Caesium 137 Isotopen (137 Cs bzw. ¹³⁷Cs) ausgesendet werden. Gammastrahlen werden von den meisten Materialien nicht abgebremst und können die meisten bei Bioprozessen verwendeten Einmalartikel durchdringen. Mikroorganismen wie Bakterien, Algen, Pilze, Viren etc. werden durch die ionisierende Strahlung durch Beschädigung ihrer Nukleinsäuren abgetötet. Gammastrahlen werden des Weiteren vom Material nicht aufgenommen und hinterlassen keine Restradioaktivität. Die Gamma-Sterilisierung von Einmalartikeln ist in einer definierten Strahlungsumgebung gut kontrollierbar und hat den Vorteil, dass weder Hitze, Feuchtigkeit, Druck oder Vakuum auf die zu sterilisierenden Gegenstände wirken. Damit stellt sie ein konstantes und vorhersehbares Sterilisierungsverfahren dar und ist vorteilhaft mit Hinblick auf Sicherheit, Zeit und Kosten.

Eine Sensorvorrichtung zum Erfassen von physikalischen oder chemischen Eigenschaften einer Flüssigkeit ist beispielsweise aus der US 2003/0227394 A1 oder der US 6,057,773 bekannt. Die bekannte Sensorvorrichtung weist in einem Gehäuse ein Sensorelement, ein Datensammelelement mit einem Datenspeicher und einem Mikrocontroller in Datenverbindung mit dem Sensorelement, eine Datenaustauschvorrichtung zur drahtlosen Weiterleitung der vom Sensorelement erfassten Daten an eine externe Einheit und eine Stromversorgungseinheit auf. Nachteilig an dieser bekannten Sensorvorrichtung ist, dass sie keine komplexen Sensorfunktionen besitzt und darüber hinaus Bauelemente aufweist, die nicht resistent gegen die Gammastrahlung sind, und unter anderem daher für die oben genannte Verwendung bei biopharmazeutischen Prozessen nicht geeignet ist.

Aus der Technologie für Sender-Empfänger-Systeme zum automatischen und berührungslosen Identifizieren und Lokalisieren von Objekten und Lebewesen mit Radiowellen (RFID) ist es bekannt, RFID Transponder einzusetzen, die resistent gegen Gammastrahlen sind. Ein Beispiel für einen derartigen RFID Transponder ist in der US 2009/0289792 A1 offenbart. Die WO 2009/120231 A1 beschreibt eine Vorrichtung zur Authentifizierung von Einwegartikeln bei der biopharmazeutischen Produktion, die einen gammastrahlungsresistenten, nichtflüchtigen FRAM-Speicherbaustein (Ferroelectric Random Access Memory) auf einem RFID Transponder aufweist, wobei die auf dem FRAM-Speicherbaustein gespeicherten Daten nach einer Gammasterilisierung des RFID Transponders erhalten bleiben.

Weiterhin ist aus der US 2014/062669 A1 ein Sensornetzwerk bekannt, in dem individuelle Sensorknoten einen Mikrocontroller aufweisen, welcher sowohl einen Gamma-resistenten FRAM und einen nicht Gamma-resistenten SRAM Speicherbaustein aufweist. Ziel ist insbesondere eine Energieersparnis gegenüber herkömmlichen nichtflüchtigen Speicherbausteinen zu erreichen.

Es ist daher die Aufgabe der vorliegenden Erfindung, eine autonome Vorrichtung zum Erfassen von Eigenschaften eines Messmediums bereitzustellen, die ohne funktionale Einschränkungen auch bei beziehungsweise nach einer Gamma-Sterilisierung in unterschiedlichsten biotechnologischen, pharmazeutischen oder chemischen Prozessen verwendet werden kann, kostengünstig herzustellen ist, eine einfache Struktur aufweist, energiesparend arbeitet sowie eine umfangreiche Messfunktionalität aufweist. Eine weitere Aufgabe der vorliegenden Erfindung ist es, ein Verfahren zum Betreiben dieser Vorrichtung bereitzustellen.

Diese Aufgabe wird durch den Gegenstand mit den Merkmalen des Anspruchs 1 gelöst. Vorteilhafte Ausgestaltungen und Ausführungsformen sind in den abhängigen Ansprüchen beschrieben.

Erfindungsgemäß weist eine autonome Vorrichtung zum Erfassen von Eigenschaften eines Messmediums einen Sensor, einen ersten Mikrocontroller und einen zweiten Mikrocontroller auf, wobei der erste Mikrocontroller einen Speicherbaustein aufweist, der nicht resistent gegen Gammastrahlung, vorzugsweise bei der Sterilisation der Vorrichtung ist, und wobei der zweite Mikrocontroller einen Speicherbaustein aufweist, der resistent gegen Gamma-strahlung, vorzugsweise bei der Sterilisation der Vorrichtung ist. Durch die Aufteilung in zwei Mikrocontroller, welche beide zusammen mit dem Sensor innerhalb des Gehäuses der Vorrichtung angeordnet sind, wird die Möglichkeit geschaffen, die autonome Vorrichtung mit einer Vielzahl von Funktionalitäten auszustatten. Durch die Resistenz des Speicherbausteins gegenüber Gammastrahlung ist gewährleistet, dass bei der Gamma-Sterilisierung der autonomen Vorrichtung die im zweiten Mikrocontroller gespeicherten Daten nicht verloren gehen. Insbesondere ist es damit möglich, dass der zweite Mikrocontroller nach der Gamma-Sterilisation den ersten Mikrocontroller mit einer entsprechenden Firmware beschreibt, die angepasste Programmbausteine für spezielle Funktionen aufweisen kann, und damit die autonome Vorrichtung zuverlässig bei biopharmazeutischen Prozessen mit Gamma-Sterilisierung verwendet werden kann. Dadurch kann bei der Auswahl der Bauelemente wie z. B. dem ersten Mikrocontroller auf günstige Komponenten zurückgegriffen werden, sodass sich insgesamt eine erhebliche Kostenersparnis ergibt, ohne dass auf bestimmte Funktionalitäten verzichtet werden muss.

Bevorzugt ist der Speicherbaustein des zweiten Mikrocontrollers ein FRAM (Ferroelectric Random Access Memory) Baustein oder auch ein RRAM (Resistive Random Access Memory) Baustein. Beide Arten von Speicherbausteinen bzw. Speicherchips sind nichtflüchtig, sparsam in Stromverbrauch und im Vergleich zu EEPROMs zuverlässiger und schneller. Neben den hier genannten FRAM und RRAM bzw. ReRAM Speicherbausteinen können auch andere dem Fachmann bekannte Speicherbausteine eingesetzt werden, die resistent gegenüber Gammastrahlen sind, wie z. B. GMRAM (Giant Magneto-Resistance Random Access Memory) oder dergleichen.

Mit weiterem Vorteil ist der erste Mikrocontroller mit einer drahtlosen Kommunikationseinrichtung versehen. Die drahtlose Kommunikationseinrichtung dient dazu, die von dem Sensor erfassten Daten an eine externe Vorrichtung vorzugsweise in Echtzeit, d. h. unmittelbar nach der Erfassung, weiterzugeben, sodass die Eigenschaften des Messmediums quasi unmittelbar und sofort extern von einer übergeordneten Vorrichtung ausgewertet und beispielsweise angezeigt werden können. Die drahtlose Kommunikationseinrichtung kann als logischer Funktionsblock in den ersten Mikrocontroller integriert sein oder über eine Hardwareschaltung mit dem ersten Mikrocontroller verbunden sein. Eine bevorzugte Art der drahtlosen Kommunikation ist die Bluetooth-Technologie. Es können aber auch andere, geeignete drahtlose Kommunikationsprotokolle und -schnittstellen in dem ersten Mikrocontroller verwendet werden.

Besonders bevorzugt ist es, wenn die Vorrichtung eine Schalteinrichtung aufweist, die bei Kontakt mit dem Messmedium einen Schaltimpuls auslöst. Eine derartige Schalteinrichtung weist mit Vorteil zwei Elektroden derart auf, dass die Elektroden bei Kontakt mit dem Messmedium den Schaltimpuls auslösen. Insbesondere ist hier eine Schalteinrichtung vorstellbar, die den Schaltimpuls auslöst, wenn sich die autonome Vorrichtung in einem flüssigen Messmedium befindet. Mit dem Schaltimpuls kann beispielsweise der zweite Mikrocontroller angeschaltet bzw. aktiviert werden, sodass eine in dem zweiten Mikrocontroller programmierte Schrittabfolge gestartet werden kann. Alternativ ist es auch möglich, dass die Schalteinrichtung den ersten Mikrocontroller einschaltet. Neben der genannten Schalteinrichtung mit zwei (Metall-) Elektroden können auch andere für denselben Zweck geeignete Schalteinrichtungen verwendet werden.

Weiter bevorzugt ist der Sensor als logischer Funktionsblock des ersten Mikrocontrollers oder des zweiten Mikrocontrollers ausgebildet. Moderne Mikrocontroller weisen üblicherweise einen Prozessor und zugleich auch Peripheriefunktionen auf, die teilweise komplexe Funktionen übernehmen können wie z. B. USB- oder Ethernet-Schnittstellen, PWM-Ausgänge, LCD-Controller oder A/D-Wandler. Mikrocontroller können ebenfalls über programmierbare digitale und/oder analoge bzw. hybride Funktionsblöcke verfügen. Unter Funktionsblock soll bei der vorliegenden Erfindung eine logische Funktion verstanden werden, die für einen bestimmten Ablauf verantwortlich ist. Ein Funktionsblock kann also ein Teil des Mikrocontrollers sein oder eigene, gegebenenfalls programmierbare Schaltungs-oder Schnittstellenkomponenten aufweisen. Der erste oder der zweite Mikrocontroller weist einen Funktionsblock auf, der für die Sensor-Funktionalität verantwortlich ist. Der Mikrocontroller kann wie oben erwähnt durch entsprechende Programmierung, d. h. Aufspielen von Firmware, mit der Funktionalität für den Sensor nach einer Löschung des Speicherbausteins beschrieben werden. Da bei einer Gamma-Sterilisierung nicht notwendigerweise alle Daten auf einem nicht dagegen resistenten Speicherbaustein unbrauchbar werden, sorgt das Aufspielen der Firmware dafür, dass die Daten des Speicherbausteins so überschrieben werden, dass die volle Funktionalität des Mikrocontrollers einschließlich aller seiner Funktionsblöcke zur Verfügung steht.

Vorteilhafterweise ist das Messmedium ein Gas, eine Flüssigkeit oder ein Granulat. Bei den meisten biopharmazeutischen Prozessen werden die autonomen Vorrichtungen gemäß der vorliegenden Erfindung in Flüssigkeiten eingesetzt.

Mit besonderem Vorteil umfasst die Funktion des Sensors das Erfassen von physikalischen und chemischen Eigenschaften des Messmediums wie beispielsweise pH-Wert, Temperatur, Viskosität, Dichte, Enzymaktivität, lonengehalt, Leitfähigkeit, Stoffkonzentration wie Sauerstoffsättigung, Härte, Druck, biologische Aktivität, Radioaktivität, Fließgeschwindigkeit und dergleichen mehr. Im Wesentlichen kann der Sensor eine beliebige physikalische oder chemische Eigenschaft des Messmediums erfassen, sofern die Technologie dafür verfügbar und im Rahmen eines programmierbaren Funktionsblocks eines Mikrocontrollers verfügbar ist. Die Sensorfunktionalität kann so programmiert sein, dass sie die Eigenschaften des Messmediums im Wesentlichen kontinuierlich oder periodisch in einem bestimmten Zeitintervall misst. Es ist auch denkbar, mehr als eine Sensorfunktionalität bereit zu stellen, d. h. pro autonomer Vorrichtung mehr als eine Eigenschaft des Messmediums zu erfassen.

Weiterhin erfindungsgemäß ist ein Verfahren zum Betrieben einer Vorrichtung zum Erfassen von Eigenschaften eines Messmediums mit folgenden Schritten: Bereitstellen einer Vorrichtung wie oben beschrieben, Durchführen einer Gamma-Sterilisierung der Vorrichtung, Einschalten des zweiten Mikrocontrollers mittels eines externen Signals, der daraufhin den ersten Mikrocontroller derart einschaltet und aktiviert, dass von dem Speicherbaustein des zweiten Mikrocontrollers auf den Speicherbaustein des ersten Mikrocontrollers eine geeignete Firmware aufgespielt wird, und Aktivieren des Sensors, sodass die Eigenschaften des Messmediums erfasst werden können. Dieses Verfahren nutzt die Eigenschaften der oben beschriebenen autonomen Vorrichtung optimal: Nach einer Gamma-Sterilisierung der gesamten autonomen Vorrichtung ist der erste Mikrocontroller nicht mehr funktionsfähig, denn die Daten auf seinem Speicherbaustein wurden durch die Bestrahlung mit Gammastrahlen zumindest teilweise beschädigt oder gelöscht. Beim Einschalten des zweiten Mikrocontrollers mittels eines externen Signals, beispielsweise durch Hineingeben der autonomen Vorrichtung in eine Flüssigkeit, wodurch ein Schaltsignal an den zweiten Mikrocontroller ausgesendet wird, wird von dem Speicherbaustein des zweiten Mikrocontrollers die Firmware auf den Speicherbaustein des ersten Mikrocontrollers aufgespielt, sodass die volle Funktionalität des ersten Mikrocontrollers wieder zur Verfügung steht. Der Speicherbaustein des ersten Mikrocontrollers kann leer sein oder auch Daten enthalten. Die Firmware enthält auch die Programmierung der entsprechenden Funktionsblöcke. Im Anschluss daran wird der Sensor bzw. der Funktionsblock des entsprechenden Mikrocontrollers aktiviert, sodass die autonome Vorrichtung die erfassten Eigenschaften des Messmediums speichern, verarbeiten und gegebenenfalls an eine externe Vorrichtung übertragen kann.

Bevorzugt erfolgt das Einschalten des zweiten Mikrocontrollers durch eine Schalteinrichtung, die bei Kontakt mit dem Messmedium einen Schaltimpuls auslöst wie bereits oben beschrieben.

Mit weiterem Vorteil wird nach Aktivieren des Sensors der erste oder der zweite Mikrocontroller ausgeschaltet. Je nachdem, welcher Mikrocontroller die Hauptfunktionen des Erfassens und des Übertragens von Daten bzw. des Abspeicherns übernimmt, kann der andere Mikrocontroller, der nach dem Aktivieren keine Funktion mehr ausübt, die dauerhaft zur Verfügung stehen muss, zeitweise oder ganz abgeschaltet werden. So kann die autonome Vorrichtung energieeffizient arbeiten, wobei auch die Lebensdauer der Bauelemente verlängert wird.

Weiterhin erfindungsgemäß ist ein System mit einem Behälter, vorzugsweise einem Zellkulturbehälter, mit mindestens einer wie oben beschriebenen autonomen Vorrichtung. Derartige Systeme werden von biopharmazeutischen Laboren für die dort durchzuführenden Prozesse benötigt. Sie umfassen im Allgemeinen einen Zellkulturbehälter mit dem Messmedium und eine Mehrzahl von autonomen Sensorvorrichtungen, um bei einem biopharmazeutischen, biotechnologischen, pharmazeutischen oder chemischen Prozess eingesetzt werden.

Vorteilhafterweise weist das System eine Auswerte- und/oder Anzeigevorrichtung auf, um die während des Prozesses erfassten Werte sofort auswerten, verarbeiten und anzeigen zu können. Damit können die Prozessparameter direkt erfasst, anzeigt und gegebenenfalls als Steuergrößen für bestimmte Prozessschritte verwendet werden.

Der Behälter ist bevorzugt als Einmal- oder Wegwerfartikel ausgebildet. Derartige das Messmedium aufnehmende Behälter müssen nach jeder Verwendung von Mikroorganismen gereinigt oder sterilisiert werden und unterliegen damit denselben Anforderungen wie die autonome Vorrichtung gemäß der vorliegenden Erfindung.

Die Erfindung wir nachfolgend anhand von Ausführungsbeispielen unter Bezugnahme auf die beigefügten Figuren erläutert, in denen:
- Fig. 1: eine schematische Darstellung einer ersten Ausführungsform der autonomen Vorrichtung der vorliegenden Erfindung zeigt; und
- Fig. 2: eine schematische Darstellung einer zweiten Ausführungsform der autonomen Vorrichtung der vorliegenden Erfindung zeigt.

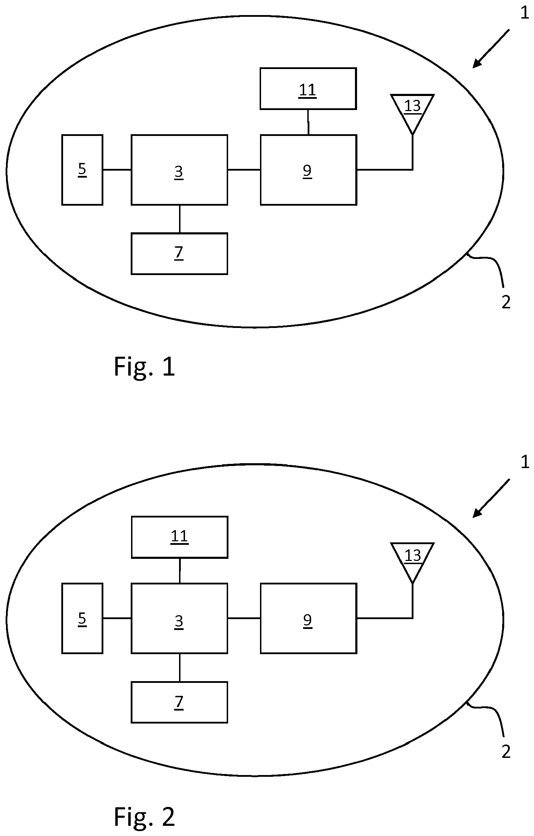

Fig. 1 zeigt eine schematische Darstellung einer ersten bevorzugten Ausführungsform der autonomen Vorrichtung gemäß der vorliegenden Erfindung, wobei die autonome Vorrichtung als sogenannter Sensorball 1 ausgebildet ist. Der Sensorball 1 weist ein wasserfestes Gehäuse 2 auf, dessen Form nicht notwendigerweise exakt der einer Kugel entsprechen muss. Die Form könnte die eines Ellipsoids, eines Würfels, eines Quaders, eines Zylinders oder einer beliebigen, auch unregelmäßigen Kombination daraus annehmen. Als Material kommt beispielsweise ein für derartige Prozesse geeignetes Kunststoffmaterial zum Einsatz, das per Spritzguss geformt ist. Das Innere des Gehäuses 2 weist eine Reihe von Bauelementen auf, darunter einen ersten Mikrocontroller 9, einen zweiten Mikrocontroller 3, eine Energieversorgungseinheit bzw. Batterie 5, eine Schalteinrichtung 7, einen Sensor 11 sowie eine drahtlose Kommunikationseinrichtung 13.

Das zentrale Element der autonomen Vorrichtung 1 ist der zweite Mikrocontroller 3, der mit der Energieversorgungseinrichtung 5, mit der Schalteinrichtung 7 und mit dem ersten Mikrocontroller 9 verbunden ist und darüber hinaus einen Speicherbaustein vom Typ FRAM (Ferroelectric Random Access Memory) aufweist, der resistent gegen Gammastrahlung bei der Sterilisation der autonomen Vorrichtung ist. Mikrocontroller mit FRAM-Speicher sind beispielsweise von den Firmen Texas Instruments oder Fujitsu erhältlich und darüber hinaus sehr sparsam im Stromverbrauch, höchst zuverlässig über einen großen Temperaturbereich und durch die FRAM-Speicher-Technologie erheblich schneller als beispielsweise Mikrocontroller mit FLASH-Speicherbausteinen. Auch eine andere Art von Gammastrahlungs-resistentem Speicher kann für den zweiten Mikrocontroller verwendet werden, beispielsweise RRAM bzw. ReRAM (Resistive Random Access Memory).

Die Energieversorgungseinrichtung 5 kann eine für derartige elektrische Schaltungen geeignete Batterie oder auch ein aufladbarer Akkumulator sein. Die Schalteinrichtung 7 ist in der bevorzugten Ausführungsform derart konfiguriert, dass sie zwei (nicht in den Figuren dargestellte) Elektroden aufweist, die an die Oberfläche des Gehäuses 2 der autonomen Vorrichtung 1 geführt sind, sodass, sobald die beiden Elektroden mit einer Flüssigkeit in Kontakt kommen, ein Schaltimpuls ausgelöst wird, der an den zweiten Mikrocontroller 3 gesendet wird. Die Schalteinrichtung 7 wird ebenfalls von der Energieversorgungseinrichtung 5 mit Energie versorgt, wobei die Leitungen über den zweiten Mikrocontroller 3 laufen. Die Schalteinrichtung 7 ist speziell auf einen geringen Stromverbrauch ausgerichtet, ihre Funktion wird weiter unten unter Bezugnahme auf die der Gesamtfunktionalität der autonomen Vorrichtung 1 beschrieben.

Der erste Mikrocontroller 9 ist mit dem zweiten Mikrocontroller 3 verbunden und weist im Gegensatz zu diesem einen Speicherbaustein auf, der nicht resistent gegen Gammastrahlen bei der Sterilisierung des Sensorballs 1 ist. Es wird deshalb bei dem ersten Mikrocontroller ein kostengünstiger handelsüblicher Speicherbaustein verwendet, z. B. ein EPROM-oder Flash-Speicherbaustein. Auch der erste Mikrocontroller 9 wird von der Energieversorgungseinrichtung 5 mit Strom versorgt, wobei auch in diesem Fall die Stromversorgung über den zweiten Mikrocontroller 3 verläuft. Der erste Mikrocontroller 9 weist eine Schnittstelle zu dem Sensor 11 auf und einen entsprechenden logischen Funktionsblock, der für die Verarbeitung der von dem Sensor 11 erfassten Signale zuständig ist. Es ist möglich, dass der erste Mikrocontroller 9 mehr als einen Sensor 11 umfasst, so dass von einem Sensorball 1 mehr als eine Eigenschaft eines Messmediums ermittelt und übertragen werden kann.

Weiterhin ist der erste Mikrocontroller 9 mit einer drahtlosen Kommunikationseinrichtung 13 verbunden, die in der bevorzugten Ausführungsform als Bluetooth-Modul, vorzugsweise auf dem Chip des ersten Mikrocontrollers 9, ausgebildet ist. Neben Bluetooth können auch andere geeignete drahtlose Datenübertragungstechnologien eingesetzt werden wie z. B. aus der IEEE 802.11 Familie oder ähnliche. Die drahtlose Kommunikationseinrichtung 13 übernimmt den Datenverkehr zwischen dem ersten Mikrocontroller 9 und einem externen Sender/Empfänger, sodass beispielsweise die von dem Sensor 11 erfassten Prozessparameter von dem ersten Mikrocontroller 9 quasi in Echtzeit nach außen übertragen und anschließend ausgewertet und verarbeitet werden können.

Nachfolgend wird die Funktionsweise der oben beschriebenen bevorzugten Ausführungsform der erfindungsgemäßen autonomen Vorrichtung und damit auch das erfindungsgemäße Verfahren zum Betreiben einer Vorrichtung zum Erfassen von Eigenschaften eines Messmediums beschrieben. Im ausgeschalteten Zustand, d. h. direkt vor der Verwendung z. B. in einem biopharmazeutischen Prozess, befindet sind die autonome Vorrichtung, gemäß der bevorzugten Ausführungsform der Sensorball 1, in dem Zustand, dass der erste Mikrocontroller 9 ausgeschaltet ist, d. h. nicht funktionsfähig ist, der zweite Mikrocontroller 3 ist in einem Schlafmodus oder Bereitschaftszustand, während dessen er auf einen externen Stimulus wartet, um in den aktiven Zustand versetzt zu werden, und die Schalteinrichtung 7 ist ebenfalls im Schlafmodus bzw. Bereitschaftszustand, sodass sie detektieren kann, wenn die entsprechenden physikalischen Voraussetzungen für das Aussenden eines Schaltimpulses vorliegen. Die beiden Peripheriefunktionen des ersten Mikrocontrollers 9, d. h. der Sensor 11 und die drahtlose Kommunikationseinrichtung 13, sind wie der erste Mikrocontroller 9 nicht eingeschaltet bzw. funktionsfähig und verbrauchen daher auch keine Energie. Dies ist der Zustand vor bzw. nach der Gamma-Sterilisierung der autonomen Vorrichtung, denn die Gammastrahlung verändert den Zustand des zweiten Mikrocontrollers 3, der Batterie 5 sowie der Schalteinrichtung 7 nicht, da der FRAM-Speicherbaustein des zweiten Mikrocontrollers 3 resistent gegen Gammastrahlen ist, ebenso wie die Schalteinrichtung 7. Im Gegensatz dazu ist der Speicherbaustein des ersten Mikrocontrollers 9 leer oder verliert bei Bestrahlung mit Gammastrahlen zumindest teilweise seine gespeicherten Daten. Dementsprechend sind auch die Schnittstellen zu dem Sensor 11 und der drahtlosen Kommunikationseinrichtung 13 nicht funktionsfähig konfiguriert. Der beschriebene Zustand ist also der Zustand vor oder nach der Reinigung bzw. Sterilisierung und damit auch der Lagerungszustand für den Sensorball 1.

Der nächste Schritt bei der bestimmungsgemäßen Verwendung der autonomen Vorrichtung bzw. des Sensorballs 1 ist die Aktivierung aus dem Lagerungszustand. Dabei wird in der bevorzugten dargestellten Ausführungsform der Sensorball 1 in eine Flüssigkeit als Messmedium eingetaucht, wodurch die Schalteinrichtung 7 mittels der beiden an der Oberfläche des Gehäuses 2 liegenden Elektroden einen Schaltimpuls auslöst und diesen Schaltimpuls auf den zweiten Mikrocontroller 3 überträgt. Der zweite Mikrocontroller erhält damit das Aktivierungssignal für seine bestimmungsgemäße Funktion, verlässt den Schlafmodus bzw. Bereitschaftszustand und schaltet den ersten Mikrocontroller 9 derart ein und aktiviert diesen, indem zunächst von dem FRAM-Speicherbaustein des zweiten Mikrocontrollers eine geeignete Firmware mit entsprechenden Programmierbefehlen auf den Speicherbaustein des ersten Mikrocontrollers 9 geschrieben bzw. aufgespielt wird. In gleicher Weise werden mit der Firmware auch die entsprechenden Programmierbefehle in die Funktionsblöcke geschrieben, die für die Schnittstellenanbindung des Sensors 11 bzw. der drahtlosen Kommunikationseinrichtung 13 an den ersten Mikrocontroller 9 zuständig sind. Nach dem vollständigen Aufspielen der Firmware einschließlich der Funktionsblockprogrammierung, das üblicherweise einige Sekunden dauert, stehen sämtliche notwendigen Funktionen des Sensorballs 1 zur Verfügung: die Messungen des Sensors 11 werden ausgeführt und die Ergebnisse der Messung vom ersten Mikrocontroller 9 über die drahtlose Kommunikationseinrichtung 13 gegebenenfalls nach außen übertragen. Gleichzeitig kann nun der zweite Mikrocontroller 3 wieder in den Schlafmodus bzw. Bereitschaftszustand versetzt werden, da seine Funktionalität für die Erfassung der Daten und für die Kommunikation der Daten nach außen nicht erforderlich ist. Damit werden Ressourcen geschont, insbesondere die Energieversorgungseinrichtung 5.

In diesem aktiven Zustand verbleibt der Sensorball 1 so lange, bis die Energieversorgungsvorrichtung 5 keine Energie mehr liefern kann oder eine weitere Sterilisierung der autonomen Vorrichtung 1 per Bestrahlung mit Gammastrahlen erfolgt. Die Gamma-Sterilisierung beschädigt bzw. löscht den nicht dagegen resistenten Speicherbaustein des ersten Mikrocontrollers 9 und damit auch sämtliche funktionale Programmierung der Schnittstellen zu dem Sensor 11 und zur drahtlosen Kommunikationseinrichtung 13. Eine Datenerfassung und eine Kommunikation der erfassten Daten ist damit nicht mehr möglich. Der Sensorball 1 befindet sich damit wieder im Lagerungszustand, der oben bereits beschrieben wurde. Damit kann der Verwendungszyklus wieder von Neuem beginnen.

Fig. 2 zeigt eine schematische Darstellung einer zweiten Ausführungsform der autonomen Vorrichtung gemäß der vorliegenden Erfindung. Da die verwendeten Komponenten im Wesentlichen dieselben sind, die bereits unter Bezugnahme auf Fig. 1 beschrieben wurden, wird an dieser Stelle auf eine Wiederholung verzichtet, und es werden nur die Unterschiede zwischen der zweiten und der ersten Ausführungsform beschrieben.

Im Unterschied zur ersten Ausführungsform ist bei der zweiten Ausführungsform der Sensor 11 nicht mit dem ersten Mikrocontroller 9, sondern mit dem zweiten Mikrocontroller 9 verbunden. Die funktionale Verarbeitung, darunter die Digitalisierung der von dem Sensor 11 erfassten Daten, findet demnach im zweiten Mikrocontroller 3 statt. Für die Funktion bzw. das Verfahren zur Verwendung der autonomen Vorrichtung hat dies zur Folge, dass der zweite Mikrocontroller 3 nach Aufspielen der Firmware auf den ersten Mikrocontroller 9 in der zweiten Ausführungsform nicht in den Schlafmodus oder Bereitschaftszustand versetzt werden darf, weil seine Aktivität für die Verarbeitung der vom Sensor 11 erfassten Signale notwendig ist. Die digitale Verarbeitung findet demnach in dem zweiten Mikrocontroller 3 statt, und die entsprechenden Daten werden anschließend auf den ersten Mikrocontroller 9 übertragen und von dort über die drahtlose Kommunikationseinrichtung 13 an die externe Datenverarbeitungseinrichtung übermittelt.

Weiterhin ist anders als bei der ersten Ausführungsform der Speicherbaustein des zweiten Mikrocontrollers 3 ein RRAM (Resistive Random Access Memory) Baustein, der ebenfalls resistent gegenüber den Einflüssen der Gammastrahlen bei der Gamma-Sterilisierung des Sensorballs 1 ist.

Ein Vorteil der zweiten Ausführungsform gegenüber der ersten Ausführungsform ist, dass der erste Mikrocontroller 9 weniger Funktion aufweist und daher kleiner dimensioniert werden kann und damit kostengünstiger ist. Dem gegenüber steht die Tatsache, dass der zweite Mikrocontroller 3 während des gesamten aktiven Messzustandes der autonomen Vorrichtung 1 eingeschaltet und aktiv sein muss.

Erfindungsgemäß ist es vorstellbar, dass eine autonome Vorrichtung eine Mischung aus den beiden oben skizzierten Ausführungsformen aufweist, d. h. dass z. B. sowohl der erste als auch der zweite Mikrocontroller jeweils über eine Sensorfunktionalität verfügen.

Mit dem Gegenstand der vorliegenden Erfindung wird eine autonome Vorrichtung zum Erfassen von Eigenschaften eines Messmediums bereitgestellt, die ohne funktionale Einschränkungen auch bei beziehungsweise nach einer Gamma-Sterilisierung in unterschiedlichsten biotechnologischen, pharmazeutischen oder chemischen Prozessen verwendet werden kann, kostengünstig herzustellen ist, eine einfache Struktur aufweist, energiesparend arbeitet sowie eine umfangreiche Messfunktionalität aufweist.

## Patentansprüche

1. Autonome Vorrichtung (1) zum Erfassen von Eigenschaften eines Messmediums, insbesondere eine Sensoreinheit zur Messung von Eigenschaften eines Messmediums, mit einem Gehäuse (2), wobei das Innere des Gehäuses (2) aufweist:
einen Sensor (11) und einen ersten Mikrocontroller (9),
wobei der erste Mikrocontroller (9) einen Speicherbaustein aufweist, der nicht resistent gegen Gammastrahlung, vorzugsweise bei der Sterilisation der Vorrichtung (1) ist,
**dadurch gekennzeichnet, dass**
das Innere des Gehäuses (2) weiterhin einen zweiten Mikrokontroller (3) aufweist,
wobei der zweite Mikrocontroller (3) einen Speicherbaustein aufweist, der resistent gegen Gammastrahlung, vorzugsweise bei der Sterilisation der Vorrichtung (1) ist.

2. Vorrichtung (1) nach Anspruch 1, **dadurch gekennzeichnet, dass** der Speicherbaustein des zweiten Mikrocontrollers (3) ein FRAM (Ferroelectric Random Access Memory) Baustein ist.

3. Vorrichtung (1) nach Anspruch 1, **dadurch gekennzeichnet, dass** der Speicherbaustein des zweiten Mikrocontrollers (3) ein RRAM (Resistive Random Access Memory) Baustein ist.

4. Vorrichtung (1) nach einem der vorhergehenden Ansprüche, **dadurch gekennzeichnet, dass** die Vorrichtung (1) als Sensorball ausgebildet ist und/oder der erste Mikrocontroller (9) mit einer drahtlosen Kommunikationseinrichtung (13) versehen ist.

5. Vorrichtung (1) nach einem der vorhergehenden Ansprüche, **dadurch gekennzeichnet, dass** sie eine Schalteinrichtung (7) aufweist, die bei Kontakt mit dem Messmedium einen Schaltimpuls auslöst.

6. Vorrichtung (1) nach Anspruch 5, **dadurch gekennzeichnet, dass** die Schalteinrichtung (7) zwei Elektroden derart aufweist, dass die Elektroden bei Kontakt mit dem Messmedium einen Schaltimpuls auslösen.

7. Vorrichtung (1) nach einem der vorhergehenden Ansprüche, **dadurch gekennzeichnet, dass** der Sensor (11) als Funktionsblock des ersten Mikrocontrollers (9) oder des zweiten Mikrocontrollers (3) ausgebildet ist.

8. Vorrichtung (1) nach einem der vorhergehenden Ansprüche, **dadurch gekennzeichnet, dass** des Messmedium ein Gas, eine Flüssigkeit oder ein Granulat ist.

9. Vorrichtung (1) nach einem der vorhergehenden Ansprüche, **dadurch gekennzeichnet, dass** die Funktion des Sensors (11) das Erfassen von physikalischen und chemischen Eigenschaften des Messmediums wie beispielsweise pH-Wert, Temperatur, Viskosität, Dichte, Enzymaktivität, lonengehalt, Leitfähigkeit, Stoffkonzentration, Härte, Druck, biologische Aktivität, Radioaktivität, Fließgeschwindigkeit und dergleichen umfasst.

10. Verfahren zum Betreiben einer Vorrichtung (1) zum Erfassen von Eigenschaften eines Messmediums mit folgenden Schritten:
Bereitstellen einer Vorrichtung (1) nach einem der vorhergehenden Ansprüche,
Durchführen einer Gammasterilisierung der Vorrichtung (1),
Einschalten des zweiten Mikrocontrollers (3) mittels eines externen Signals, der daraufhin den ersten Mikrocontroller (9) derart einschaltet und aktiviert, dass von dem Speicherbaustein des zweiten Mikrocontrollers (3) auf den Speicherbaustein des ersten Mikrocontrollers (9) eine geeignete Firmware aufgespielt wird, und Aktivieren des Sensors (11), so dass die Eigenschaften des Messmediums erfasst werden können.

11. Verfahren nach Anspruch 10, **dadurch gekennzeichnet, dass** das Einschalten durch eine Schalteinrichtung (7) erfolgt, die bei Kontakt mit dem Messmedium einen Schaltimpuls auslöst.

12. Verfahren nach Anspruch 10, **dadurch gekennzeichnet, dass** nach Aktivieren des Sensors (11) der erste (9) oder der zweite Mikrocontroller (3) ausgeschaltet wird.

13. System mit einem Behälter, vorzugsweise einem Zellkulturbehälter, mit mindestens einer Vorrichtung (1) nach einem der Ansprüche 1 bis 9.

14. System nach Anspruch 13, **dadurch gekennzeichnet, dass** es eine Auswerte-und/oder Anzeigevorrichtung aufweist.

15. System nach Anspruch 13 oder 14, **dadurch gekennzeichnet, dass** der Behälter als Einmal- oder Wegwerfartikel ausgebildet ist.

## Claims

1. Autonomous device (1) for detecting the characteristics of a measurement medium, more particularly a sensor unit for measuring the properties of a measurement medium, having
a housing (2) wherein the inside of the housing (2) comprises:
a sensor (11) and a first microcontroller (9),
wherein the first microcontroller (9) has a memory chip which is not resistant to gamma rays, preferably during the sterilization of the device (1)
**characterised in that** the inside of the housing (2) further comprises a second microcontroller (3) wherein the second microcontroller (3) comprises a memory chip which is resistant to gamma rays, preferably during sterilization of the device (1).

2. Device (1) according to claim 1 **characterised in that** the memory chip of the second microcontroller (3) is a FRAM (ferroelectric random-access memory) chip.

3. Device (1) according to claim 1 **characterised in that** the memory chip of the second microcontroller (3) is a RRAM (resistive random-access memory) chip.

4. Device (1) according to one of the preceding claims **characterised in that** the device (1) is configured as a sensor ball and/or the first microcontroller (9) is provided with a wireless communications device (13).

5. Device (1) according to one of the preceding claims, **characterised in that** it has a switching mechanism (7) which triggers a switching pulse on contact with the measurement medium.

6. Device (1) according to claim 5 **characterised in that** the switching mechanism (7) comprises two electrodes in such a way that the electrodes trigger a switching pulse on contact with the measurement medium.

7. Device (1) according to one of the preceding claims **characterised in that** the sensor (11) is designed as a function block of the first microcontroller (9) or of the second microcontroller (3).

8. Device (1) according to one of the preceding claims **characterised in that** the measurement medium is a gas, a fluid or a granulated material.

9. Device (1) according to one of the preceding claims **characterised in that** the function of the sensor (11) comprises detecting physical and chemical properties of the measurement medium such as by way of example the pH-value, temperature, viscosity, density, enzyme activity, ion content, conductivity, material concentration, hardness, pressure, biological activity, radioactivity, flow speed and the like.

10. Method for operating a device (1) for detecting characteristics of a
measurement medium, comprising the following steps:
providing a device (1) according to one of the preceding claims,
performing gamma sterilization of the device (1),
switching on the second microcontroller (3) by means of an external signal which then switches on and activates the first microcontroller (9) so that a suitable firmware is loaded from the memory chip of the second microcontroller (3) to the memory chip of the first microcontroller (9), and
activating the sensor (11) so that the characteristics of the measurement medium can be detected.

11. Method according to claim 10 **characterised in that** the switch-on is carried out by a switching mechanism (7) which triggers a switching pulse on contact with the measurement medium.

12. Method according to claim 10 **characterised in that** after activation of the sensor (11) the first microcontroller (9) or the second microcontroller (3) is switched off.

13. System having a container, preferably a cell culture container, with at least one device (1) according to one of claims 1 to 9.

14. System according to claim 13 **characterised in that** it has an evaluation and/or indicator device.

15. System according to claim 13 or 14 **characterised in that** the container is configured as a single-use or disposable article.

## Revendications

1. Dispositif autonome (1) pour la détection de propriétés d'un milieu de mesure, en particulier une unité de capteur pour la mesure de propriétés d'un milieu de mesure, avec un boîtier (2), dans lequel l'intérieur du boîtier (2) présente :
un capteur (11) et un premier microcontrôleur (9),
dans lequel le premier microcontrôleur (9) présente un module de mémoire qui n'est pas résistant au rayonnement gamma, de préférence lors de la stérilisation du dispositif (1),
**caractérisé en ce que**
l'intérieur du boîtier (2) présente en outre un second microcontrôleur (3),
dans lequel le second microcontrôleur (3) présente un module de mémoire qui est résistant au rayonnement gamma, de préférence lors de la stérilisation du dispositif (1).

2. Dispositif (1) selon la revendication 1, **caractérisé en ce que** le module de mémoire du second microcontrôleur (3) est un module FRAM (Ferroelectric Random Access Memory).

3. Dispositif (1) selon la revendication 1, **caractérisé en ce que** le module de mémoire du second microcontrôleur (3) est un module RRAM (Resistive Random Access Memory).

4. Dispositif (1) selon l'une des revendications précédentes, **caractérisé en ce que** le dispositif (1) est réalisé en tant que bille de capteur et/ou le premier microcontrôleur (9) est pourvu d'un équipement de communication (13) sans fil.

5. Dispositif (1) selon l'une des revendications précédentes, **caractérisé en ce qu'**il présente un équipement de commutation (7) qui déclenche, lors du contact avec le milieu de mesure, une impulsion de commutation.

6. Dispositif (1) selon la revendication 5, **caractérisé en ce que** l'équipement de commutation (7) présente deux électrodes de telle manière que les électrodes déclenchent, lors du contact avec le milieu de mesure, une impulsion de commutation.

7. Dispositif (1) selon l'une des revendications précédentes, **caractérisé en ce que** le capteur (11) est réalisé en tant que bloc fonctionnel du premier microcontrôleur (9) ou du second microcontrôleur (3).

8. Dispositif (1) selon l'une des revendications précédentes, **caractérisé en ce que** le milieu de mesure est un gaz, un liquide ou un granulat.

9. Dispositif (1) selon l'une des revendications précédentes, **caractérisé en ce que** la fonction du capteur (11) comporte la détection de propriétés physiques et chimiques du milieu de mesure telles que valeur pH, température, viscosité, densité, activité enzymatique, teneur en ions, conductivité, concentration de substance, dureté, pression, activité biologique, radioactivité, vitesse d'écoulement et similaires.

10. Procédé de fonctionnement d'un dispositif (1) pour la détection de propriétés d'un milieu de mesure avec les étapes suivantes :
la mise à disposition d'un dispositif (1) selon l'une des revendications précédentes,
la réalisation d'une stérilisation gamma du dispositif (1),
la mise sous tension du second microcontrôleur (3) au moyen d'un signal externe qui met sous tension et active subséquemment le premier microcontrôleur (9) de telle manière qu'un microprogramme approprié soit chargé du module de mémoire du second microcontrôleur (3) sur le module de mémoire du premier microcontrôleur (9), et
l'activation du capteur (11) de sorte que les propriétés du milieu de mesure puissent être détectées.

11. Procédé selon la revendication 10, **caractérisé en ce que** la mise sous tension est effectuée par un équipement de commutation (7) qui déclenche, lors du contact avec le milieu de mesure, une impulsion de commutation.

12. Procédé selon la revendication 10, **caractérisé en ce qu'**après l'activation du capteur (11) le premier (9) ou le second microcontrôleur (3) est mis hors tension.

13. Système avec un récipient, de préférence un récipient de culture cellulaire, avec au moins un dispositif (1) selon l'une des revendications 1 à 9.

14. Système selon la revendication 13, **caractérisé en ce qu'**il présente un dispositif d'évaluation et/ou d'affichage.

15. Système selon la revendication 13 ou 14, **caractérisé en ce que** le récipient est réalisé en tant qu'article à usage unique ou jetable.
